Europäisches Patentamt

European Patent Office

Office européen des brevets

(19)

(11) **EP 1 721 540 A1**

(12) **EUROPEAN PATENT APPLICATION**
published in accordance with Art. 158(3) EPC

(43) Date of publication:
**15.11.2006 Bulletin 2006/46**

(51) Int Cl.:
***A45D 7/06*** (2006.01)

(21) Application number: **05720094.1**

(22) Date of filing: **28.02.2005**

(86) International application number:
**PCT/JP2005/003822**

(87) International publication number:
**WO 2005/084483 (15.09.2005 Gazette 2005/37)**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**LV**

(30) Priority: **05.03.2004 JP 2004002879**

(71) Applicant: **Sakakibara, Itsuo
Nagoya-shi
Aichi 4660812 (JP)**

(72) Inventor: **Sakakibara, Itsuo
Nagoya-shi
Aichi 4660812 (JP)**

(74) Representative: **TBK-Patent
Bavariaring 4-6
80336 München (DE)**

(54) **PERMANENT TREATMENT METHOD**

(57) A permanent treatment method of the present invention is a method, which has been invented in order to provide a permanent treatment process, which can maintain a straightened-hair state of hair for a long time. The treatment method of the present invention is equipped with: a permeating step of adhering a permanent treatment liquid, which includes thioglycolic acid, down to a position, which is adjacent to the hair root of hair, thereby permeating the permanent treatment liquid into the hair root; and a shaping step of pulling the hair while warming the hair, which is in such a state that the permanent treatment liquid is adhered thereon, to a predetermined temperature, thereby shaping it to straight hair. In accordance with this permanent method, hair, which has extended anew since the permanent treatment, turns into straight hair.

**Fig. 2**

Printed by Jouve, 75001 PARIS (FR)

**Description**

TECHNICAL FIELD

The present invention relates to a permanent treatment method for shaping hair to desired shapes, and relates to a permanent treatment method for shaping hair to straight hair particularly.

BACKGROUND ART

As for a permanent treatment method for shaping curve-shaped hair to straight hair, various methods have been known which are referred to as straight permanent or curly-hair correction. These methods are a method in which a permanent treatment liquid, which includes a reducing agent, and an oxidizing agent are acted onto hair to shape the hair to straight hair. In the present description, a permanent treatment method for shaping hair to straight hair is referred to as a permanent treatment method. Moreover, in the present description, curve-shaped hair, which has been referred to as wavy hair or curly hair in general, is referred to as curly hair, and hair, whose shape is not curved, is referred to as straight hair.

As the permanent treatment liquid, which has been employed in the permanent treatment method, those which include thioglycolic acid or its salts as a reducing agent, have been known.

When the permanent treatment liquid is adhered onto hair, by means of hydrogen ions, which generate from thioglycolic acid, cystine, which constitutes keratin, the major component of hair, is reduced to open up the S-S bond of cystine. By means of this, the elasticity of hair becomes small to give flexibility to it so that it becomes possible to shape it to desired shapes. In this state, by compressing or pulling the hair, the hair is elongated physically. Thereafter, by acting the oxidizing agent onto the hair, the S-S bond closes in such a state that the hair is elongated, and thereby the hair is shaped to a straight-hair shape. As for the oxidizing agent used herein, bromate, such as sodium bromate, perborate, such as sodium perborate, hydrogen peroxide, oxygen in air, and the like, have been employed (for instance, Patent Literature Nos. 1-2).

In Patent Literature No. 1, there is set forth a permanent treatment method in which a permanent treatment liquid, which includes a reducing agent, is acted onto hair and thereafter the hair is compressed, thereby sustaining a straight-hair state of curly hair for a long period of time.

Moreover, in Patent Literature No. 2, there is set forth a method in which a permanent treatment liquid, which includes a reducing agent, is acted onto hair and thereafter the hair is compressed while warming it, thereby shaping the hair to straight hair. Further, in this Patent Literature No. 2, there is set forth to the effect that, lest the permanent treatment liquid adheres onto the scalp or onto the hair root, it is preferable to use a jelly-shaped permanent treatment liquid.

By the way, among the hair, the hair shaft, a part which is exposed to the outside of the hair follicle, is shaped to straight hair by the conventional permanent treatment methods set forth in these patent publications. However, since hair, which has extended anew since the treatments, still continues to be curly hair, it has been necessary to regularly perform a permanent treatment to it in order to maintain the straight-hair state of the hair.

[Patent Literature No. 1]
Japanese Unexamined Patent Publication (KOKAI) No. 09-132, 515
[Patent Literature No. 2]
Domestic Re-publication of PCT International Publication for Patent Applications No. 00-064,298

DISCLOSURE OF THE INVENTION

(Assignment to be solved by the Invention)

The present invention is one which has been done while taking the aforementioned circumstance into consideration, and it is an object to provide a permanent treatment method which can maintain a straight-hair state of hair for a long time.

(Means for Solving the Assignment)

A permanent treatment method of the present invention is characterized in that: a permeating step of adhering a permanent treatment liquid, which includes thioglycolic acid, down to a position, which is adjacent to the hair root of hair, thereby permeating the permanent treatment liquid into the hair root; and a shaping step of pulling the hair while warming the hair, which is in such a state that the permanent treatment liquid is adhered thereon, to a predetermined temperature, thereby shaping it to straight hair.

In the aforementioned permanent treatment method of the present invention, between said permeating step and said shaping step, it is preferable to be equipped with a treatment-liquid absorbing step of sprinkling a treatment powder, which adsorbs or absorbs said permanent treatment liquid, onto at least a part of said hair to transfer said permanent treatment liquid from said hair to the treatment powder, thereby stopping the shaping temporarily.

In the permanent treatment method of the present invention, the concentration of said thioglycolic acid, which is included in said permanent treatment liquid, can preferably be 7% by weight or less of the entirety of said permanent treatment liquid.

Moreover, it is preferable that said permanent treatment liquid can further include cysteine; and the sum of the concentration of said thioglycolic acid and the concentration of the cysteine can be 7% by weight of the entirety of said permanent treatment liquid.

In the case of carrying out the treatment-liquid absorbing step, it is preferable that the treatment powder can be at least one member of hydrophilic organic powders and hydrophilic inorganic powders.

Said permeating step can preferably be carried out while warming said hair.

Said permeating step can preferably be such that said hair can be left for 20 minutes or more after adhering said permanent treatment liquid to it.

Said permeating step can preferably be such that said hair, which is in such a state that said permanent liquid is adhered thereon, can be vibrated with ultrasonic vibrating means.

Said shaping step can preferably be such that said hair, which is in such a state that said permanent treatment liquid is adhered thereon, or said hair on which said treatment powder is sprinkled, can be vibrated with ultrasonic vibrating means.

Said ultrasonic vibrating means can preferably be equipped with an ultrasonic oscillator and an ultrasonic-waves absorbing plate, which is disposed to face the oscillator with a variable interval provided therebetween; and said vibrating can preferably be carried out while holding said hair, which is in such a state that saidpermanent treatment liquid is adhered thereon, or said hair on which said treatment powder is sprinkled, between the ultrasonic oscillator and the absorbing plate.

Said ultrasonic vibrating means can preferably be equipped with an ultrasonic oscillator and an ultrasonic-waves reflector plate, which is disposed to face the oscillator so as to make the interval with respect to the oscillator an integer multiple of 1/2 of the wavelength of an ultrasonic wave, which the ultrasonic oscillator generates; and said vibrating can preferably be carried out while holding said hair, which is in such a state that said permanent treatment liquid is adhered thereon, or said hair on which said treatment powder is sprinkled, between the ultrasonic oscillator and the reflector plate.

Said predetermined temperature in said shaping step can preferably be 100 °C or more.

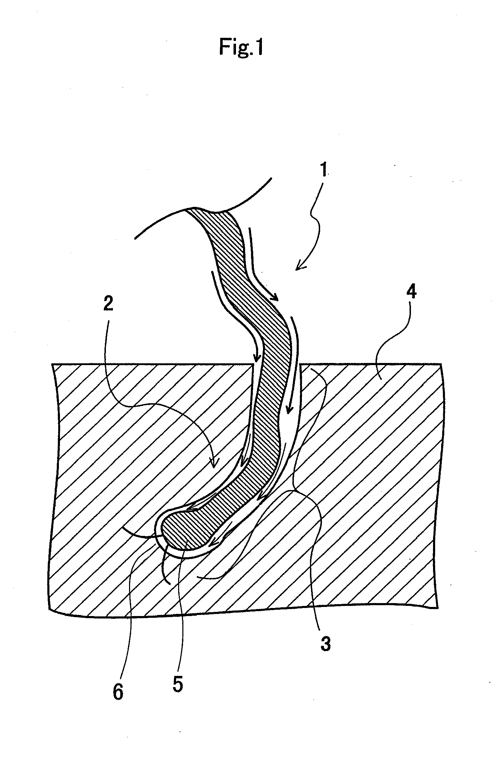

In accordance with the permanent treatment method of the present invention, hair, which has extended anew since the permanent treatment, turns into a straight-hair state. The reason has not been clear yet, however, it is believed to result from the following actions. A cross-sectional view for schematically illustrating a hair-root portion in curly hair is shown in Fig. 1.

Among hair 1, a hair root 3 is a portion, which is positioned in a hair follicle 2, a part of which is completed by being constituted of a scalp 4. In curly hair, it has been known that this hair root 3 is curbed as an arc shape. When adhering a permanent treatment liquid onto a portion adjacent to the hair root 3 among this hair 1, the permanent treatment liquid permeates into the hair root 3 within the hair follicle 2. When the permanent treatment liquid permeates into the hair root 3, since thioglycolic acid, which is included in the permanent treatment liquid, acts to the hair root 3, the elasticity of the hair root 3 becomes small, and additionally flexibility is given to the hair root 3.

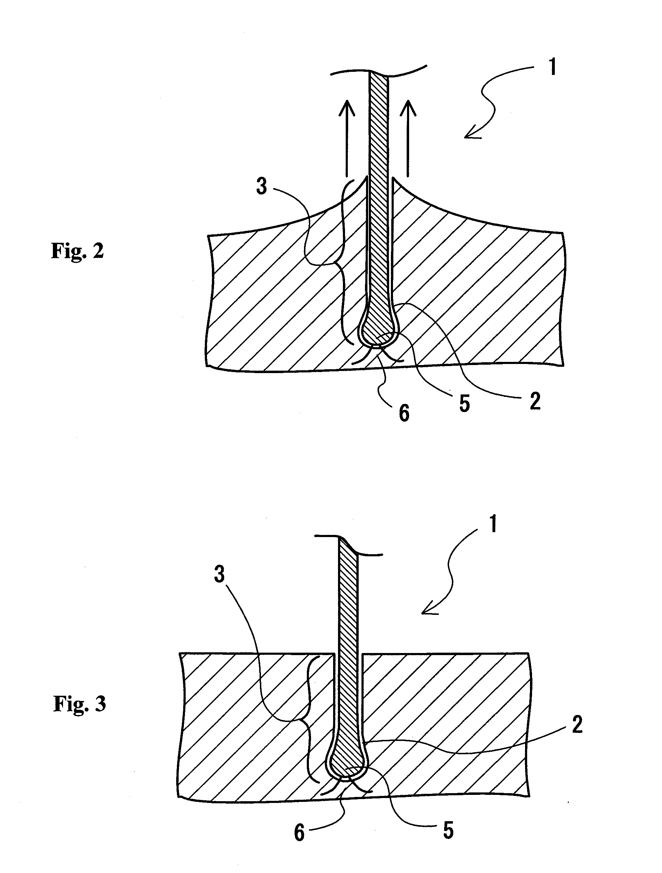

In this state, as shown in Fig. 2, by pulling the hair 1 while warming it, the hair 1 is shaped to a straight-hair shape as shown in Fig. 3. And, by shaping the hair 1 to a straight-hair shape, it is believed that the hair 3 too is shaped to a straight configuration.

Here, among the hair root 3, a hair base end 5 is a part, which contacts with a hair papilla 6, the bottom of the hair follicle 2, the elongation of the hair 1 occurs because of the fact that the cell division occurs here. Therefore, since the hair base end 5 receives a certain action because of the fact that the hair root 3 is shaped to a straight configuration, it is believed that the hair 1, which extends after the permanent treatment, turns into a straight-hair shape.

Here, in the conventional permanent treatment methods, the permanent treatment liquid is adhered onto the portions alone, which are separated from the hair root, among the hair. It is because of the fact that, in the permanent treatment method for shaping hair to straight hair, since external forces, such as pulling and compressing, are applied to the hair, there is a fear that the hair might be cut off upon pulling if the permanent treatment liquid is adhered onto a portion, which is close to the hair root among the hair, to make the elasticity of this portion small.

Therefore, in the conventional permanent methods, the permanent treatment liquid does not permeate down to the hair root, and accordingly hair, which has extended anew since the permanent treatment, has been still kept to be as curly hair. In the permanent treatment method of the present invention, because of the fact that the permanent treatment liquid is permeated down to the hair root and the hair is pulled while warming it, it is possible to turn the hair, which extends after the permanent treatment, into a straight-hair shape, as described above.

In the aforementioned permanent treatment method of the present invention, between said permeating step and said shaping step, it is preferable to be equipped with a treatment-liquid absorbing step of sprinkling a treatment powder, which adsorbs or absorbs said permanent treatment liquid, onto at least a part of said hair to transfer said permanent treatment liquid from said hair to the treatment powder, thereby stopping the shaping temporarily. When carrying out the shaping step to the hair after this treatment-liquid absorbing step, first of all, the treatment powder, which adsorbs or absorbs the permanent treatment liquid, is warmed. And, because of the fact that it is warmed, the reduction-reaction rate of cystine in the portion of the hair with which the treatment powder contacts becomes large, and accordingly the shaping of the hair is resumed. Moreover, by means of the fact that the treatment powder is warmed so that the permanent treatment liquid bleeds out of the treatment powder to transfer to the hair again, the shaping is resumed.

Here, it has been known in general that curly hair curves greatly in a wet state; and that the curving becomes small in a dry state. Therefore, in hair in a wet state, since the apparent difference between the portion, in which the shaping has developed so that the curving disappears or becomes small, and the portion, in which the shaping is insufficient so that the hair is still kept to be curved, becomes small, it has been very difficult to distinguish the shaped state. However, in the case of carrying out the treatment-liquid absorbing step between the permeating step and the shaping step, the permanent treatment liquid, which is adhered on the hair, is adsorbed or absorbed into the treatment powder, and accordingly the hair is turned into a substantially dry state. Consequently, since it is possible to visually judge between the portion, in which the shaping develops sufficiently, and the portion, in which the shaping is insufficient, among the hair, with ease, an advantage is available in that it is possible to shape the hair to a straight-hair shape more securely. Because of the fact that the hair is shaped to a straight-hair shape securely, the hair root is also turned into a straight state securely, and accordingly hair, which extends after the permanent treatment, turns into straight hair more securely.

Moreover, when the shaping step is carried out to the hair after this treatment-liquid absorbing step as aforementioned, first of all, the treatment powder, which adsorbs or absorbs the permanent treatment liquid, is warmed, however, it does not occur that water vapor blows up even when the warming temperature exceeds 200 °C. When carrying out the shaping step without the treatment-liquid absorbing step, the treatment liquid, which is adhered on the hair, causes a kind of water-vapor explosion so that it is dangerous.

In the permanent treatment method of the present invention, in the case of using the permanent treatment liquid whose thioglycolic-acid concentration is 7% by weight or less of the entire permanent treatment liquid, an advantage is available in that cut-off hair becomes less likely to occur upon shaping.

Moreover, in the case of further compounding cysteine with the permanent treatment liquid so as to make the sum of the thioglycolic-acid concentration and the cysteine concentration 7% by weight of the entire permanent treatment liquid, an advantage is available in that cut-off hair becomes less likely to occur upon shaping.

It is preferable that the treatment powder used in the treatment-liquid absorbing step can be at least one member of hydrophillic organic powders and hydrophillic inorganic powders. Thioglycolic acid ($C_2H_4O_2S$), which is included in the permanent treatment liquid, is a water-soluble liquid, which is referred to as mercaptoacetic acid or mercaptoethanoic acid, and the permanent treatment liquid is an aqueous solution in which thioglycolic acid is dissolved into water. Consequently, by means of the hydrophillic treatment powder, the permanent treatment liquid is adsorbed or absorbed very well.

In the case of carrying out the permeating step while warming the hair, the permanent treatment liquid becomes likely to permeate into the hair root, and accordingly it is possible to turn hair, which extends after the permanent treatment, into a straight-hair state more securely.

In the case of leaving the hair, on which the permanent treatment liquid is adhered, for 20 minutes or more in the permeating step, since the permeation of the permanent treatment liquid into the hair is done more securely, it is possible to turn hair, which extends after the permanent treatment, into a straight-hair shape more securely.

When ultrasonic vibrating the hair, on which the permanent treatment liquid is adhered, in the permeating step, since the permanent treatment liquid goes deep into the hair follicle so that it permeates down to the hair papilla of the hair, it is possible to turn hair, which extends after the permanent treatment, into a straight-hair shape more securely.

When vibrating the hair, which is in such a state the permanent treatment liquid is adhered thereon (without the permanent-liquid absorbing step) , or the hair on which the treatment powder is sprinkled (with the permanent-liquid absorbing step) with ultrasonic vibrating means in the shaping step, since the permanent treatment liquid goes deep into the hair follicle so that it permeates down to the hair papilla of the hair, it is possible to turn hair, which extends after the permanent treatment, into a straight-hair shape more securely.

Moreover, when ultrasonic vibrating the hair while holding the hair, which is in such a state that permanent liquid is adhered thereon, or the hair on which the treatment powder is sprinkled, between an ultrasonic oscillator and an absorbing plate, which absorbs ultrasonic waves, in the permeating step or the shaping step, since an ultrasonic wave, which generates from the ultrasonic oscillator, is absorbed by the absorbing plate, there is no canceling action by means of reflected wave, and accordingly the ultrasonic wave acts on the hair effectively.

Moreover, when ultrasonic vibrating the hair while holding the hair, which is in such a state that permanent liquid is adhered thereon, or the hair on which the treatment powder is sprinkled, between an ultrasonic oscillator and an ultrasonic-waves reflector plate, which is disposed to face the oscillator so as to make an interval with respect to the oscillator an integer multiple of 1/2 of the wavelength of an ultrasonic wave, which the ultrasonic oscillator generates, in the permeating step or the shaping step, since the ultrasonic wave, which has generated from the ultrasonic oscillator, and ultrasonic waves reflected at the reflector plate interfere with each other to intensify with each other, the permeation action of the treatment liquid into the hair enlarges.

Moreover, in the case of adapting the warming temperature in the shaping step to be 100 °C or more, hair, which extends after the permanent treatment, turns into a straight-hair shape more securely. Note that the "warming temperature" set forth herein is referred to as the temperature of means per se for warming the hair. For example, in the case of warming the hair with a hair iron, the instance of adapting a portion, among the hair iron, which is brought into contact with the hair to be 100 °C or more is referred to as "adapting the warming temperature of the hair to be 100 °C or more." Since the higher the warming temperature is the more the shaping action enlarges, it is desired to adapt it to be 220 °C approximately. When carrying out the shaping step while warming it without the treatment-liquid absorbing step, the treatment liquid, which is adhered on the hair, causes a kind of water-vapor explosion so that it has been dangerous. However, because of the fact that it goes through the treatment-liquid absorbing step, even when the warming temperature exceeds 200 °C, since the treatment powder absorbs the permanent treatment liquid to turn it into a dry state so that no water vapor blows up.

It is advisable as well to carry out the shaping step dividedly twice; to carry out a pre-shaping step, the first shaping step, between the permeating step and the treatment-liquid absorbing step; and to carry out a post-shaping step, the second shaping step, after the treatment-liquid absorbing step. Because of the fact that a portion, among the hair, which has not been shaped completely by mean of the pre-shaping step is detected; and the portion is shaped once again by means of the post-shaping step, the hair is shaped to straight hair more securely. And, hair, which extends after the permanent treatment, too, turns into straight hair more securely.

## BRIEF DESCRIPTION OF THE DRAWINGS

Fig. 1 is a cross-sectional diagram for schematically illustrating a hair-root portion among curly hair.

Fig. 2 is a cross-sectional diagram for schematically illustrating the appearance of performing a shaping step to curly hair.

Fig. 3 is a cross-sectional diagram for schematically illustrating hair, which is after having been permanent treated by a permanent treatment method of the present invention.

Fig. 4 is a photograph for showing hair in which 4 months had elapsed since a permanent treatment method of comparative example.

Fig. 5 is a major-part-enlarged photograph for showing the hair in which 4 months had elapsed since the permanent treatment method of comparative example.

Fig. 6 is a photograph for showing hair in which 4 months had elapsed since a permanent treatment method of example.

Fig. 7 is a major-part-enlarged photograph for showing the hair in which 4 months had elapsed since the permanent treatment method of example.

Fig. 8 is a side diagram of ultrasonic vibrating means, and Fig. 9 is the A-A cross-sectional diagram of Fig. 8.

## BEST MODE FOR CARRYING OUT THE INVENTION

In the permanent treatment method of the present invention, the permanent treatment liquid is such that it is possible to employ known ones which include thioglycolic acid. The larger the concentration of thioglycolic acid included in the permanent treatment liquid is, such an advantage is available that the more the time required for the permanent treatment is shortened. Moreover, the smaller the concentration of thioglycolic acid is, such an advantage is available that the less likely cut-off hair becomes to occur upon shaping.

The concentration of thioglycolic acid included in the permanent treatment liquid can preferably be 7% by weight or less with respect to the permanent treatment liquid. As described above, it is because of suppressing cut-off hair upon shaping. Note that, in the case where the concentration of thioglycolic acid in the permanent treatment liquid exceeds 7% by weight, cut-off hair becomes likely to occur during shaping, however, in this case as well, hair, which has extended since the permanent treatment, turns into straight hair. Therefore, the concentration of thioglycolic acid in the permanent treatment liquid can exceed 7% by weight.

The concentration of thioglycolic acid in the permanent treatment liquid can desirably be 4.5% by weight or more and 7% by weight or less. It is adapted to be 4.5% by weight or more, because the higher the concentration of thioglycolic acid is the more securely the shaping can be done in a shorter period of time. Note that, even when the concentration of thioglycolic acid is less than 4.5% by weight, it is possible to shape the hair to a straight-hair shape securely by increasing the amount of the permanent treatment liquid to be adhered onto the hair or prolonging the time taken for the permeating step and shaping step. And, in this case as well, hair, which has extended since the permanent treatment step, turns into straight hair.

In the permanent treatment method of the present invention, it is possible to further compound cysteine with the permanent treatment liquid. Cysteine, similarly to thioglycolic acid, produces hydrogen ions and works as a reducing agent for reducing cystine. Since the reducing action of cystine by means of cysteine is weaker than thioglycolic acid, by using cysteine with thioglycolic acid combinedly, such an effect is available that the hair becomes less likely to be cut off upon shaping. In this case, the sum of the concentration of thioglycolic acid and the concentration of cysteine can preferably be 7% by weight of the entirety of the permanent treatment liquid.

Moreover, in all of the cases as well, the pH of the permanent treatment liquid can preferably fall in the range of 4.5-9.6, and can desirably be 8-9 approximately. Although thioglycolic acid is acidic, it is possible to set up the pH of the permanent treatment liquid in the aforementioned range by compounding a known alkaline agent, which is represented by ammonia or monoethanolamine, with the permanent treatment liquid. Note that, in the case of employing the salt of thioglycolic acid, such as an ammonium salt of thioglycolic acid, for instance, an alkaline agent cannot be compounded therewith in particular.

In the permeating step, the part of the hair onto which the permanent treatment liquid is adhered is such that the more adjacent position to the hair root is the more preferable it is.

For example, in the case of adhering the permanent treatment liquid onto a position close to the hair root to such an extent that it adheres onto the scalp, the permanent treatment liquid goes into the hair follicle directly or transmits through the scalp or hair shaft to permeate into the hair root.

Moreover, since the permanent treatment liquid moves in the hair-root direction by means of the capillary phenomenon between hairs neighboring with each other, even in the case of adhering the permanent treatment liquid onto the hair root, a portion coming out from the hair follicle, alone, the permanent liquid goes into the inside of the hair follicle to permeate into the hair root.

Further, since the hair is in a tube shape, depending on the amount of the permanent treatment liquid adhered onto the hair or the adhesion part, and the like, the permanent treatment liquid, which has adhered on the hair-shaft surface, permeates into the inside of the hair and reaches the hair root.

In all of the cases as well, the permanent treatment liquid is such that it is preferable to adhere it onto such an extent of position that, among the hair shaft, a portion around the hair follicle is wetted by means of the permanent treatment liquid.

The permeating step is such that it is preferable to carry it out while warming the hair, however, the temperature at this moment can preferably be 30-45 °C. Since the reaction rate that cystine is reduced by means of thioglycol depends on the temperature, in the case where a large temperature unevenness exists depending on the parts of the hair, there might arise cases where the hair cannot be shaped homogeneously. However, in the case of carrying out the permeating step while warming the hair, since the temperature unevenness is reduced so that the cystine of the hair is reduced without unevenness, it is possible to shape the hair homogeneously in the shaping step. Moreover, in this permeating step, by covering the hair with a cap or wrapping material, and the like, it is possible as well to reduce the temperature unevenness much more.

Note that, as described above, this permeating step is such that it is preferable to leave the hair for a while after the permanent treatment liquid is adhered onto it, and it is desirable to leave it for 20 minutes or more. It is because of reducing cystine in the hair more securely by permeating it thereinto while taking time.

The permeating step and/or the shaping step can preferably be carried out while vibrating it with ultrasonic vibrating means, however, the frequency of ultrasonic wave in this instance can preferably fall in the range of 20-100 kHz. When the power of ultrasonic wave is higher, the treatment liquid permeates into the hair root even in a shorter vibrating time; and when it is lower, it is advisable to lengthen the vibrating time. For example, it is possible to use ultrasonic vibrating means whose frequency is 20 kHz and power is 10-20 W.

Let the frequency of ultrasonic wave be f, and the acoustic velocity be v, an ultrasonic wave from an oscillator and an ultrasonic wave reflected at a reflector plate intensify with each other when the interval L between the oscillator and the reflector plate is:

$$L = n(v/f) \qquad (1)$$

Here, n is an integer, and can be 1, 2, 3, ⋯. For example, let f = 20 kHz, and n = 1, since v = 300 m/sec, it becomes L = 7.5 mm. Namely, in the case of vibrating it with a 20-kHz-frequency-ultrasonic vibrating means, it is possible to act ultrasonic wave strongly onto the hair by holding the hair between the oscillator and the reflector plate so that the interval between them becomes 7.5 mm.

In the shaping step, the temperature for warming the hair, which is in such a state that the permanent treatment liquid is adhered thereon without the treatment-liquid absorbing step, can desirably fall in the range of 160-180 °C. At this moment, since the permanent treatment liquid is adhered on the hair, a temperature, which acts onto the hair actually, becomes a temperature of being the warming temperature or less, and accordingly it does not cause so-called water-vapor explosion. When the hair-warming temperature falls in this range, it is possible to shape the hair to a sufficient straight-hair shape in a short time relatively. In the shaping step, in the case of warming it after the treatment-liquid absorbing step, it can preferably fall in the range of 200-250 °C. Since the treatment liquid is absorbed into the treatment powder so that the hair is turned into a dry state, it does not result in water-vapor explosion. When the hair-warming temperature falls in this range, it is possible to shape the hair to a sufficient straight-hair shape in a short time relatively.

In the shaping step, the larger the force for pulling the hair the more preferable it is, however, when the pulling time is longer, the force for pulling the hair can be smaller relatively, for instance. Here, the force for pulling the hair can be 0.5 kg/cm$^2$ or more, and can more preferably be 0.8 kg/cm$^2$ or more. And, it can desirably be 1.0 kg/cm$^2$ or more. Note that the force for pulling the hair set forth herein is judged by the extent that the scalp is pulled. For example, the case where the scalp is pulled to the same extent as that a 10-kg weight is hung from hairs, which are grown in 10 square cm, to pull them down is referred to as "the hair is pulled with 1 kg/cm$^2$."

In the case of carrying out the treatment-liquid absorbing step between the permeating step and the shaping step, as for a treatment powder to be sprinkled onto the hair, it can preferably be at least one member of hydrophilic organic powders and hydrophilic inorganic powders, as described above. Among them, as for hydrophilic organic powder, sugars, alcohols, carboxylic acids, esters, and the like, which have hydrophilic groups, such as a hydroxyl group, a carboxyl group and an ester group, can be used preferably. Note that, as for these organic powders, those which are solid at ordinary temperature can be used. As for hydrophilic inorganic powders, it is possible to cite various metallic oxides. Among them, viscosity minerals, silica, alumina, and so forth, whose specific surface area is large can be used especially preferably.

In the permanent treatment method of the present invention, it is possible to re-oxidize cystine, which has been reduced, by acting an oxidizing agent onto the hair after the shaping step, depending on the composition of the permanent treatment liquid, and the like. Note that, in the case of selecting one, which is referred to as a mono-bath type which uses oxygen in air as an oxidizing agent, as the permanent treatment liquid, it is advisable not to act an oxidizing agent to it separately.

Hereinafter, the permanent treatment method of the present invention will be explained while naming examples.

First of all, to the hairs of a subject who had curly hairs, a permanent treatment was performed by means of a permanent treatment method of a comparative example, which will be explained hereinafter. Note that, in this subject's hairs, silver gray hairs were mixed partially.

(Comparative Example)

(Cystine Reduction Step)

Onto the hairs, which had been shampooed and from which a hair dressing, the oil contents, and the like, had been removed, a permanent treatment liquid was adhered. This permanent treatment liquid was one which included 4.0%-by-weight thioglycolic acid, 1.5%-by-weight cysteine hydrochloride, 1.5%-by-weight monoethanolamine, 1.5%-by-weight ammonia, 1.5%-by-weight cetanol, and 1.0%-by-weight lauryl chloride trimethylammonium, when the entirety was taken as 100%.

At this moment, the permanent treatment liquid was adhered onto the leading-end-side portions by 1 cm or more beyond the scalp alone, among the hairs, so as not to adhere it onto the hair roots. The entire hairs, on which the permanent treatment liquid was adhered, were covered with a cap, which was made from resin, and were left for 20 minutes as they were while warming them with a lamp, which was set at 40 °C.

(Pre-shaping Step)

After the completion of the cystine reduction step, the hairs, which were in such a state that the permanent treatment liquid was adhered thereon, were pulled with a therapeutist's hands of while warming them using a hair iron. The hair iron used herein was one whose portions to be brought into contact with the hairs were heated to 80 °C.

First of all, the hairs were subjected to blocking for every grasp. A block was held with the hair iron, and was pulled while sliding the hair iron from a position around the scalp to the hair-ends side in the state of being held. The completion when the hair iron had slid from a position around the scalp toward the hair-ends side was regarded as one-time pulling. The one-time pulling was carried out while taking about 5 seconds, and the pulling was carried out three times in total. The force for pulling the hairs at this moment was 0.5 kg/cm$^2$ approximately. Regarding the hairs of the other blocks as well, the pulling was likewise carried out three times each, thereby shaping the entire hairs. Note that the subject's hairs when the present comparative example was carried out were about 14 cm.

(Treatment-liquid Absorbing Step)

After the completion of the pre-shaping step, onto the hairs which were in such a state that the permanent treatment liquid was adhered thereon, a treatment powder, which was composed of CMC (carboxymethylcellulose) was sprinkled, to such an extent that the surface of the hairs appeared to be dry. By means of this step, the permanent treatment liquid was absorbed into the treatment powder, and accordingly the shaping was interrupted temporarily.

(Post-shaping Step)

After the treatment-liquid absorbing step, the shaped state of the hairs was judged visually. The portions of the hairs, into which the shaping was not done sufficiently and which did not turn in straight hair, were selected, and the shaping of the hairs was carried out by the same method as the above-described pre-shaping step. In this post-shaping step, with respect to one block of the hairs, the pulling was carried out twice each. By means of this post-shaping step, the hairs, which were not shaped completely in the pre-shaping step, were shaped, and the entire hairs were shaped to straight hair homogeneously.

(Oxidizing Step)

After the post-shaping step, an oxidizing agent, which included 10%-by-weight sodium bromate with respect to the entire oxidizing agent, was acted to the hairs. By means of this oxidizing step, since cystine, which was oxidized by means of thioglycolic acid in the above-described permeating step, was re-oxidized, the shaped state of the hairs was fixed as a straight-hair shape.

(Post-treatment Step)

After completing from the permeating step to the oxidizing step, the hairs were shampooed to remove the permanent treatment liquid, the treatment powder and the oxidizing agent. Thereafter, the hairs were dried with a hair dryer.

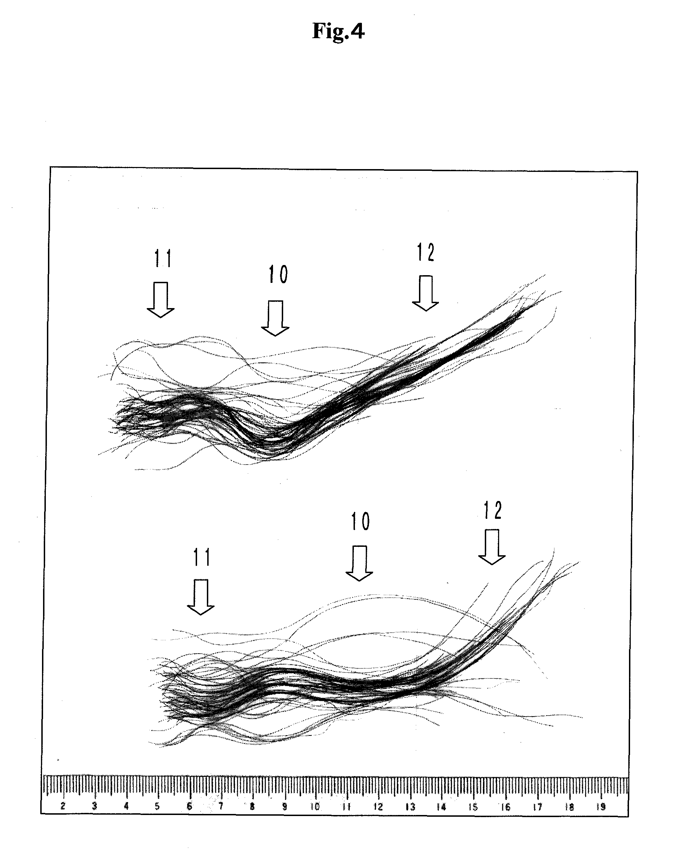

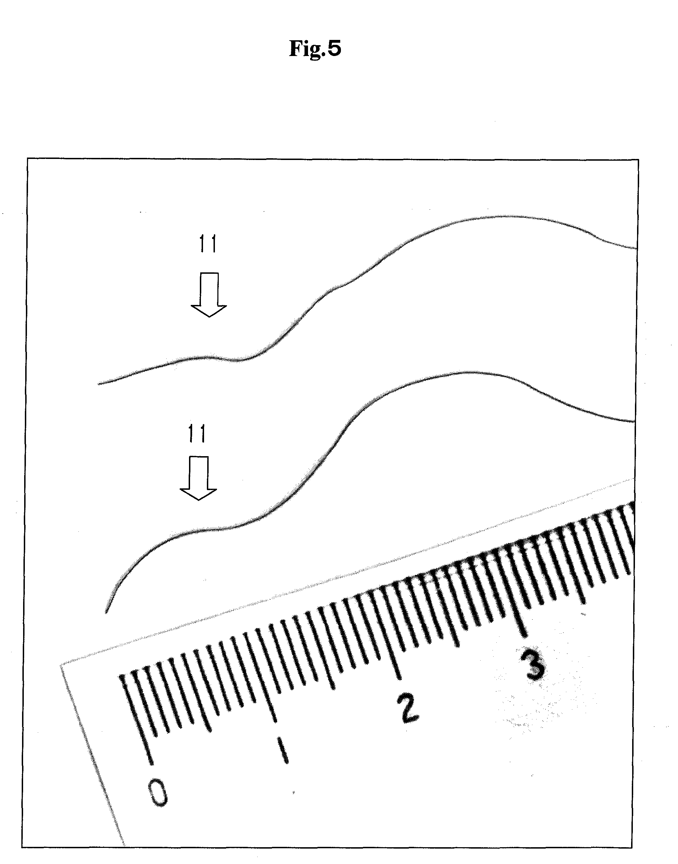

By means of the above permanent treatment, the subject's hairs were shaped to straight hair. After 4 months had elapsed since the permanent treatment of the comparative example, the subject's hairs were collected. And, the state of hairs, which had extended anew since the permanent treatment of the comparative example, was determined visually. A photograph for showing the subject's hairs after 4 months had lapsed since the permanent treatment of the comparative example is illustrated in Fig. 4. Moreover, a major-portion-enlargement photograph of the hairs is illustrated in Fig. 5. As illustrated in Fig. 4, among the collected hairs 10, the portions 11, which were the near side to the hair roots, turned into a curly-hair shape, and the portions 12, which were the far side from the hair roots, turned into a straight-hair shape. The straight-hair-shaped portions 12 are portions to which the permanent treatment of the comparative example was performed, and the curly-hair-shaped portions 11 were portions which had extended anew since the permanent treatment of the comparative example. From this result, it was understood that, by the permanent method of the comparative example, that is, a conventional permanent treatment method, the hair-shaft portions were shaped to straight hair, but the portions, which had extended anew since then, were still kept to be curly hair. Note that the hairs were those to which a coloring treatment was performed immediately before being collected.

(Example No. 1)

After 4 months had elapsed since the permanent treatment of the above-described comparative example, a permanent treatment by means of a permanent treatment method of Example No. 1 was performed to the right half of the identical subject's hairs.

(Permeating Step)

Onto the hairs, which had been shampooed and from which a hairdressing agent, the oil contents, and the like, had been removed, a permanent treatment liquid was adhered. This permanent treatment liquid was the same one as the permanent treatment liquid used in the comparative example.

At this moment, the permanent treatment liquid was adhered onto the entire hairs to such an extent that the scalp was wetted. The hairs, on which the permanent treatment liquid was adhered, were covered with a cap, which was made from resin, and were left for 20 minutes as they were while warming them with a lamp, which was set at 40 °C.

(Treatment-liquid Absorbing Step)

After the permeating step, onto the hairs which were in such a state that the permanent treatment liquid was adhered thereon, a treatment powder, which was composed of CMC (carboxymethylcellulose) was sprinkled, to such an extent that the surface of the hairs appeared to be dry. By means of this step, the permanent treatment liquid was absorbed into the treatment powder, and accordingly the shaping was interrupted temporarily.

(Shaping Step)

After the permanent-liquid absorbing step, the hairs, which were in such a state that CMC, which absorbed the permanent treatment liquid, was adhered thereon, were pulled with a therapeutist' s hands while warming them using a hair iron. The hair iron used herein was one whose portions to be brought into contact with the hairs were heated to 160 °C.

First of all, the hairs were subjected to blocking for every grasp. A block was held with the hair iron, and was pulled while sliding the hair iron from a position around the scalp to the hair-ends side in the state of being held. The completion when the hair iron had slid from a position around the scalp toward the hair-ends side was regarded as one-time pulling. The one-time pulling was carried out while taking about 5 seconds, and the pulling was carried out four times in total. The force for pulling the hairs at this moment was 1 kg/cm$^2$ approximately. Regarding the hairs of the other blocks as well, the pulling was carried out four times each, thereby shaping the entire hairs. Note that the subject's hairs when the present comparative example was likewise carried out were about 18 cm.

(Oxidizing Step)

After the shaping step, an oxidizing agent, which was the same as the comparative example, was acted to the hairs, thereby fixing the shaped state of the hairs as a straight-hair shape.

(Post-treatment Step)

After completing from the permeating step to the oxidizing step, the hairs were shampooed to remove the permanent treatment liquid, the treatment powder and the oxidizing agent. Thereafter, the hairs were dried with a hair dryer.

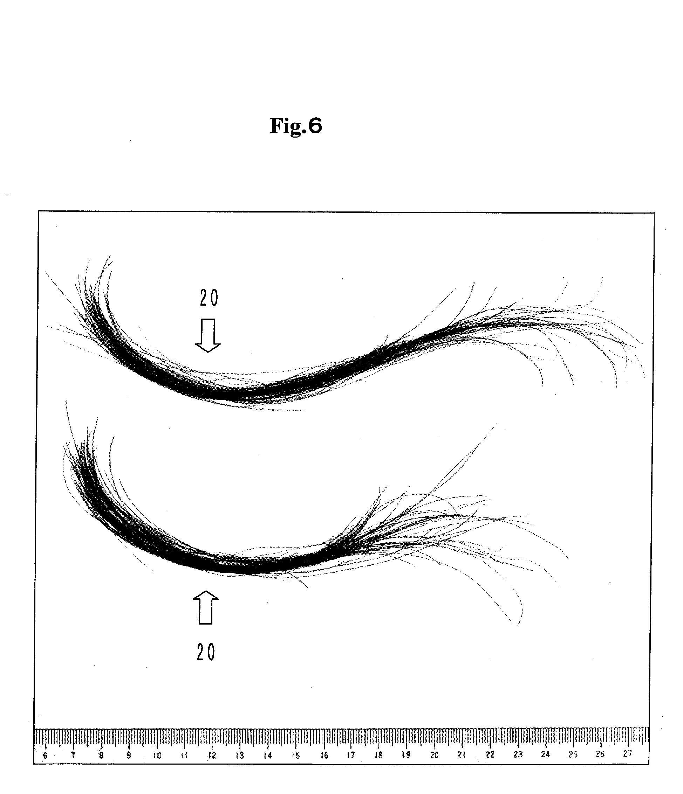

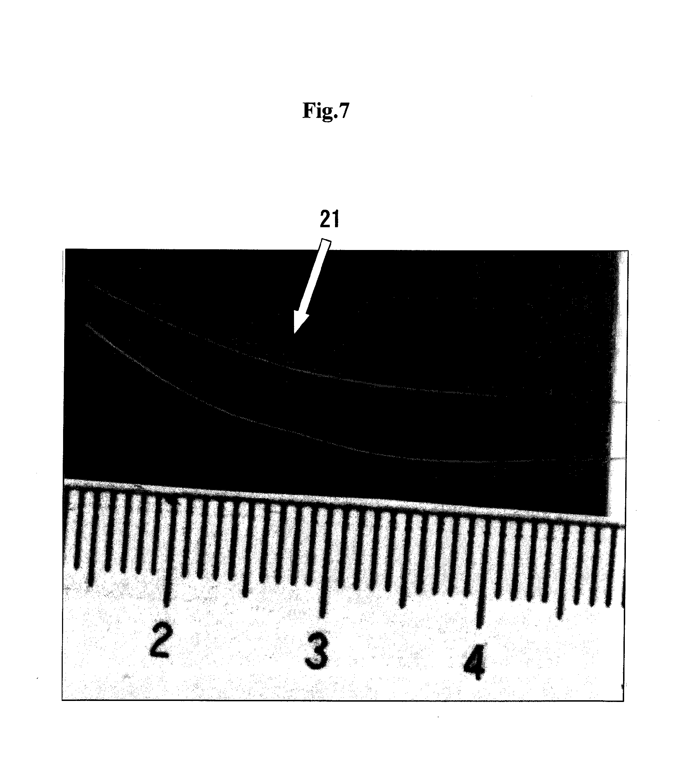

By means of the above permanent treatment method, the subject's hairs were shaped to straight hair, and the portions, which were shaped to straight hair, were hair-dyed. After 4 months had elapsed since the permanent treatment of the present example, the subject's hairs were collected. And, the state of hairs, which had extended anew since the permanent treatment of the present example, was determined visually. A photograph for showing the subject's hairs after 4 months had elapsed since the permanent treatment of the present example is illustrated in Fig. 6. Moreover, a major-portion-enlargement photograph of the hairs is illustrated in Fig. 7. As illustrated in Fig. 6, about 80% of the collected hairs 20, including the newly-extended portions as well, turned into a straight-hair shape. Although Fig. 7 illustrates white portions 21, the portions which had extended since the permanent treatment of the present example, it is understood that they are in a straight-hair shape. From this result, it was understood that, in the permanent treatment method of the present example, the hair-root portions are shaped to straight hair, and additionally the portions, which have extended anew thereafter, turn into straight hair.

(Example No. 2)

After 4 months had elapsed since the permanent treatment of the above-described comparative example, a permanent treatment by means of a permanent treatment method of Example No. 2 was performed to the left half of the identical subj ect' s hairs. Since the present example is the same as Example No. 1 except for the permeating step and shaping step, only the permeating step and shaping step will be hereinafter explained.

(Permeating Step)

Onto the hairs, which had been shampooed and from which a hairdressing agent, the oil contents, and the like, had been removed, a permanent treatment liquid was adhered. This permanent treatment liquid was the same one as the permanent treatment liquid used in the comparative example.

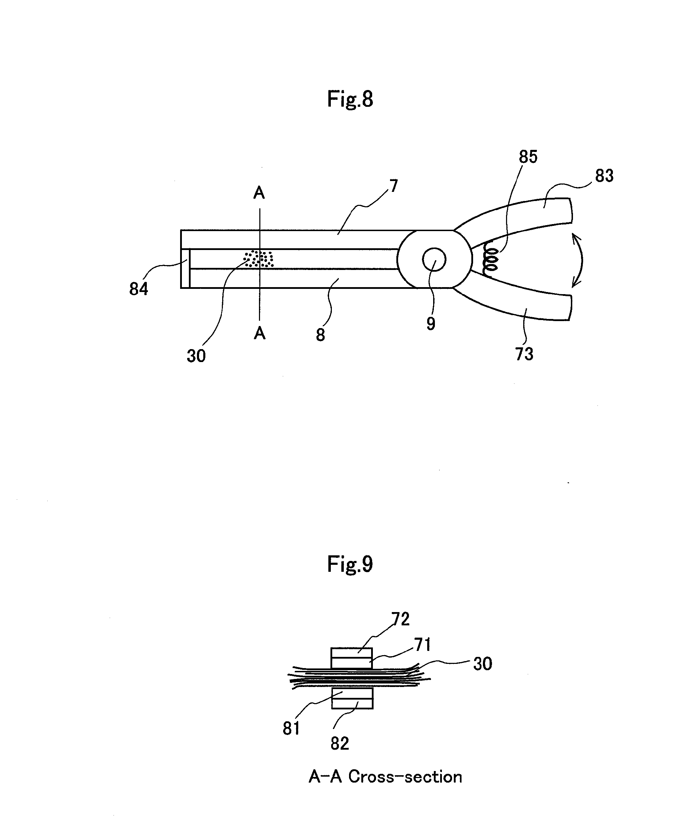

At this moment, the permanent treatment liquid was adhered onto the entire hairs to such an extent that the scalp was wetted. The hairs, on which the permanent treatment liquid was adhered, were vibrated with ultrasonic vibrating means illustrated in Fig. 8 and Fig. 9. The vibrating means is equipped with arms 7, 8, which can rotate around a hinge shaft 9 against the urging force of a spring 85, and an ultrasonic oscillator 71 is attached to the arm 7 on one of whose sides faces the arm 8. Moreover, a reflector plate 81 is attached to one of the sides of the arm 8 facing the arm 7, and a detachable stopper 84 is attached to the leading end. 72 and 82 are heaters, 30 is hairs, 73 is the grip of the arm 7, and 83 is the grip of the arm 8. From the ultrasonic oscillator 71, an ultrasonic wave whose frequency is 20 KHz and power is 16 W is launched out. The height of a stopper 83 is set up so that the space between the arms 7 and 8 becomes 7.5 mm in such a state that the arm 7 is brought into contact with the stopper 83 (the state of Fig. 8). For every grasp, the hairs 30, which were subjected to blocking, were pinched with the arms 7 and 8, were rotated about the hinge shaft 9 to bring the arm 7 into contact with the stopper 83, and were vibrated for 30 seconds while setting the heaters 72 and 82 at 40 °C. Regarding the hairs of the other blocks as well, they were vibrated similarly.

Note that the ultrasonic vibrating means is not limited to the above descriptions (Fig. 8 and Fig. 9), and it is advisable that 81 can be adapted to be an ultrasonic oscillator, for instance. Alternatively, it is advisable that 81 can be adapted to be an ultrasonic-waves absorbing plate.

(Shaping Step)

After the permanent-liquid absorbing step, the hairs, which were in such a state that CMC, which absorbed the permanent treatment liquid, was adhered thereon, were pulled with a therapeutist' s hands while warming and vibrating them using the ultrasonic vibrating means of Fig. 8 and Fig. 9. Note that the vibrating means in this case is such that the stopper 83 was removed, and accordingly can hold the hairs 30 powerfully. Moreover, the heaters 72 and 82 were set at 220 °C.

First of all, the hairs were subjected to blocking for every grasp. A block was held with the vibrating means, and was pulled while sliding the vibrating means from a position around the scalp to the hair-ends side in the state of being held. The completion when the vibrating means had slid from a position around the scalp toward the hair-ends side was regarded as one-time pulling. The one-time pulling was carried out while taking about 5 seconds, and the pulling was carried out four times in total. The force for pulling the hairs at this moment was 1 kg/cm$^2$ approximately. Regarding the hairs of the other blocks as well, the pulling was likewise carried out four times each, thereby shaping the entire hairs. Note that the subject's hairs when the present comparative example was carried out were about 18 cm.

By means of the permanent treatment of the present example, the subject's hairs were shaped to straight hair, and the portions, which were shaped to straight hair, were hair-dyed. After 4 months had elapsed since the permanent treatment of the present example, the subject's hairs were collected. And, the state of hairs, which had extended anew since the permanent treatment of the present example, was determined visually. 95-100% of the collected hairs, including the newly-extended portions as well, turned into a straight-hair shape. From this result, it was understood that, in the permanent treatment method of the present example, the hair-root portions are shaped to straight hair, and additionally the portions, which have extended anew thereafter, turn into straight hair.

**Claims**

1. A permanent treatment method, being **characterized in that** it is equipped with: a permeating step of adhering a permanent treatment liquid, which includes thioglycolic acid, down to a position, which is adjacent to the hair root of hair, thereby permeating the permanent treatment liquid into the hair root; and
a shaping step of pulling the hair while warming the hair, which is in such a state that the permanent treatment liquid is adhered thereon, to a predetermined temperature, thereby shaping it to straight hair.

2. The permanent treatment method set forth in claim 1, being **characterized in that**, between said permeating step and said shaping step, it is equipped with a treatment-liquid absorbing step of sprinkling a treatment powder, which adsorbs or absorbs saidpermanent treatment liquid, onto at least a part of said hair to transfer said permanent treatment liquid from said hair to the treatment powder, thereby stopping the shaping temporarily.

3. The permanent treatment method set forth in claim 1 or 2, being **characterized in that** the concentration of said thioglycolic acid, which is included in said permanent treatment liquid, is 7% by weight or less of the entirety of said permanent treatment liquid.

4. The permanent treatment method set forth in claim 1 or 2, being **characterized in that** said permanent treatment liquid further includes cysteine, and the sum of the concentration of said thioglycolic acid and the concentration of the cysteine is 7% by weight of the entirety of said permanent treatment liquid.

5. The permanent treatment method set forth in claim 2, being **characterized in that** said treatment powder is at least one member of hydrophilic organic powders and hydrophilic inorganic powders.

6. The permanent treatment method set forth in either one claim of claims 1 through 5, being **characterized in that** said permeating step includes warming said hair, which is in such a state that said permanent treatment liquid is adhered thereon.

7. The permanent treatment method set forth in either one claim of claims 1 through 6, being **characterized in that** said permeating step includes leaving said hair, which is in such a state that said permanent treatment liquid is adhered thereon, for 20 minutes or more.

8. The permanent treatment method set forth in either one claim of claims 1 through 7, being **characterized in that** said permeating step includes vibrating said hair, which is in such a state that said permanent treatment liquid is adhered thereon, with ultrasonic vibrating means.

9. The permanent treatment method set forth in either one claim of claims 1 through 8, being **characterized in that** said shaping step includes vibrating said hair, which is in such a state that said permanent treatment liquid is adhered thereon, or said hair on which said treatment powder is sprinkled, with ultrasonic vibrating means.

10. The permanent treatment method set forth in claim 8 or 9, being **characterized in that** said ultrasonic vibrating means is equipped with an ultrasonic oscillator and an ultrasonic-waves absorbing plate, which is disposed to face the oscillator with a variable interval provided therebetween; and said vibrating is carried out while holding said hair, which is in such a state that said permanent treatment liquid is adhered thereon, or said hair on which said treatment powder is sprinkled, between the ultrasonic oscillator and the absorbing plate.

11. The permanent treatment method set forth in claim 8 or 9, being **characterized in that** said ultrasonic vibrating means is equipped with an ultrasonic oscillator and an ultrasonic-wavesreflector plate, which is disposed to face the oscillator so as to make the interval with respect to the oscillator an integer multiple of 1/2 of the wavelength of an ultrasonic wave, which the ultrasonic oscillator generates; and said vibrating is carried out while holding said hair, which is in such a state that said permanent treatment liquid is adhered thereon, or said hair on which said treatment powder is sprinkled, between the ultrasonic oscillator and the reflector plate.

12. The permanent treatment method set forth in either one claim of claims 1 through 10, being **characterized in that** said predetermined temperature in said shaping step is 100 °C or more.

## Fig.1

Fig. 2

Fig. 3

# Fig.4

**Fig.5**

**Fig.6**

**Fig.7**

21

## Fig.8

## Fig.9

A–A Cross–section

## INTERNATIONAL SEARCH REPORT

| International application No. |
|---|
| PCT/JP2005/003822 |

A.  CLASSIFICATION OF SUBJECT MATTER
   Int.Cl⁷  A45D7/06, A61K7/09

According to International Patent Classification (IPC) or to both national classification and IPC

B.  FIELDS SEARCHED

Minimum documentation searched (classification system followed by classification symbols)
   Int.Cl⁷  A45D7/06

Documentation searched other than minimum documentation to the extent that such documents are included in the fields searched
   Jitsuyo Shinan Koho          1922-1996    Jitsuyo Shinan Toroku Koho    1996-2005
   Kokai Jitsuyo Shinan Koho    1971-2005    Toroku Jitsuyo Shinan Koho    1994-2005

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y<br>A | JP 2000-256146 A  (Kabushiki Kaisha Mirubon),<br>19 September, 2000 (19.09.00),<br>Full text; all drawings<br>Full text; all drawings<br>(Family: none) | 1-10,12<br>11 |
| Y<br>A | JP 2001-72557 A  (Nobutaka OKA),<br>21 March, 2001 (21.03.01),<br>Full text; all drawings<br>Full text; all drawings<br>(Family: none) | 1-10,12<br>11 |
| Y<br>A | JP 2003-159115 A  (Wella AG.),<br>03 June, 2003 (03.06.03),<br>Full text; all drawings<br>Full text; all drawings<br>& EP 1302124 A2          & DE 10150765 A | 2-10,12<br>1,11 |

☒  Further documents are listed in the continuation of Box C.          ☐  See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    28 March, 2005 (28.03.05) | Date of mailing of the international search report<br>    12 April, 2005 (12.04.05) |
|---|---|
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2005/003822 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y<br>A | JP 2000-37222 A  (Matsushita Electric Works,<br>Ltd.),<br>08 February, 2000 (08.02.00),<br>Full text; all drawings<br>Full text; all drawings<br>(Family: none) | 8-10,12<br>1-7,11 |
| Y<br>A | JP 2000-139545 A  (Matsushita Electric Works,<br>Ltd.),<br>23 May, 2000 (23.05.00),<br>Full text; all drawings<br>Full text; all drawings<br>(Family: none) | 9-10,12<br>1-8,11 |
| Y<br>A | JP 2001-299432 A  (Kabushiki Kaisha Kikuhoshi),<br>30 October, 2001 (30.10.01),<br>Full text; all drawings<br>Full text; all drawings<br>(Family: none) | 10,12<br>1-9,11 |

Form PCT/ISA/210 (continuation of second sheet) (January 2004)

**EP 1 721 540 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 9132515 A
- WO 00064298 A